# EUROPÄISCHE PATENTSCHRIFT

(11) **EP 1 051 389 B1**
(45) Veröffentlichungstag und Bekanntmachung des Hinweises auf die Patenterteilung: **23.04.2003**
(21) Anmeldenummer: 98965854.7
(22) Anmeldetag: 17.12.1998
(51) Int. Cl.: C07C 213/04, C07C 29/128, C08G 65/10, C08G 73/02

(54) **VERFAHREN ZUR HERSTELLUNG OXALKYLIERTER AMINE ODER ALKOHOLE**
METHOD FOR PRODUCING OXALKYLATED AMINES OR ALCOHOLS
PROCEDE DE PREPARATION D'AMINES OU D'ALCOOLS OXALKYLES

(30) Priorität: 23.12.1997 DE 19757709
(43) Veröffentlichungstag der Anmeldung: 15.11.2000
(73) Patentinhaber: BASF AKTIENGESELLSCHAFT, 67056 Ludwigshafen (DE)
(72) Erfinder: MOHR, Jürgen, D-67269 Grünstadt (DE); DOCKNER, Toni, D-67149 Meckenheim (DE)
(74) Vertreter: Isenbruck, Günter, Dr.
(86) Internationale Anmeldenummer: EP9808302
(87) Internationale Veröffentlichungsnummer: WO99033783

(56) Entgegenhaltungen:
- WO-A-91/15441
- WO-A-94/09055
- DE-A- 19 502 970
- US-A- 3 313 846

## Beschreibung

Die Erfindung betrifft ein Verfahren zur Herstellung oxalkylierter Amine oder Alkohole oder deren Gemischen, bei dem ein Amin oder ein Gemisch aus zwei oder mehr Aminen, oder ein Alkohol oder ein Gemisch aus zwei oder mehr Alkoholen, oder ein Gemisch aus einem oder mehreren Aminen und einem oder mehreren Alkoholen, mit einem Alkylenoxid, oder einem Gemisch aus zwei oder mehr verschiedenen Alkylenoxiden, in einer oder mehreren aufeinanderfolgenden Reaktionsstufen umgesetzt wird.

Die Umsetzungsprodukte von Aminen oder Alkoholen mit Alkylenoxiden sind als oberflächen- und grenzflächenaktive Verbindungen bekannt und kommen in verschiedensten technischen Bereichen zur Anwendung. Insbesondere sind dies beispielsweise Wasch- und Reinigungsmittel, Körperpflegemittel oder technische Anwendungen, beispielsweise der Einsatz als Emulgatoren, Dispergatoren, Emulsionsspalter oder Dispersionsspalter, oder als Zwischenprodukte, Verdicker oder Schmierstoffe.

Aus dem Stand der Technik bekannte Verfahren zur Oxalkylierung von Aminen werden in der Regel ein- oder zweistufig durchgeführt, während zur Oxalkylierung von Alkoholen in der Regel einstufige Verfahren bevorzugt werden.

So beschreibt beispielsweise die DE-A 195 44 739 ein Verfahren zur Herstellung von oxalkylierten Polyethyleniminen durch Oxalkylierung von Polyethyleniminen in einer oder zwei Verfahrensstufen zu Reaktionsprodukten, die 1 bis 200 mol Alkylenoxidgruppen pro Aminogruppe im Polyethylenimin enthalten. Bei einstufiger Verfahrensführung werden wasserfreie Polyethylenimine und 1 bis 20 mol-%, bezogen auf Polyethylenimine, mindestens einer wasserfreien Base vorgelegt oder wäßrige Lösungen dieser Stoffe getrocknet und nach dem Entfernen des gesamten Wassers bei Temperaturen von mehr als 135 bis 150°C mit mindestens einem Alkylenoxid umgesetzt. Bei der zweistufigen Verfahrensführung wird in der ersten Verfahrensstufe Polyethylenimin bei Temperaturen von 80 bis 100°C mit 0,7 bis 0,9 mol, bezogen auf 1 mol Aminogruppen im Polymerisat, mindestens eines Alkylenoxids in wäßriger Lösung umgesetzt, und in der zweiten Verfahrensstufe das in der ersten Verfahrensstufe erhaltene Reaktionsprodukt in Gegenwart von 1 bis 20 mol-%, bezogen auf Polyethylenimin, eines alkalischen Katalysators in Abwesenheit von Wasser bei Temperaturen von 120 bis 150°C mit mindestens einem Alkylenoxid zu oxalkylierten Polyethyleniminen umgesetzt, die 1 bis 200 mol Alkylenoxidgruppen pro Aminogruppe im Polyethylenimin enthalten. Hellfarbige oder fast farblose Produkte werden gemäß der Druckschrift erst dann erhalten, wenn mit einer hohen Katalysatorkonzentration gearbeitet wird. Eine Aussage über die olfaktorischen Eigenschaften des entstehenden Produkts läßt sich der Druckschrift nicht entnehmen.

Auch ist bereits bekannt, hydrophobierte Hydrotalcite als Katalysatoren für die Ethoxylierung bzw. Propoxylierung von Verbindungen mit aktiven H-Atomen oder Fettsäureestern zu verwenden, wobei sich eine enge Homologenverteilung der Alkoxylierungsprodukte ergibt (WO 91/15441).

Bekannt ist auch ein Verfahren zur diskontinuierlichen Herstellung von Polyxoxibutylenpolyoxialkylenglykolen durch Copolymerisation von Tetrahydrofuran mit 1,2-Alkylenoxiden in Gegenwart von Verbindungen mit reaktivem Wasserstoff am säurebehandelten, calcinierten Kaolinfestbettkatalysator. Durch während der Reaktion konstant gehaltener Alkylenoxid-Konzentration von unter 1 Gew.-% im Polymerisationsansatz gelingt es dabei, die Bildung cyclischer Ether weitgehend zu unterdrücken und ein sehr eng verteiltes Polymerisat zu erhalten (DEE 195 02 970 A1).

Weiterhin offenbart die internationale Patentanmeldung WO 94/09055 ein Verfahren zur Polymerisation von Oxiranen, Oxetanen, Oxepanen, Trioxanen und Tetrahydrofuranen an speziellen Metallverbindungen.

Ferner ist auch eine Methode zur Bildung Polymerer mit terminalen Carboxylgruppen beschrieben, wobei Olefinoxide, die weniger als 5 Kohlenstoff-Atome enthalten, mit Wasser an einem Katalysator, der im wesentlichen aus einer Kombination eines Zinksalzes einer Carbonsäure ausgewählt aus der Gruppe Ameisensäure, Essigsäure und Glykolsäure, und Aluminiumisopropylat besteht, umgesetzt werden (US 3,313,846).

Vorschläge für geeignete Reaktionsbedingungen bei der Herstellung von grenzflächenaktiven Oxalkylierungsprodukten finden sich beispielsweise bei N. Schönfeldt, Grenzflächenaktive Äthylenoxid-Addukte, Wissenschaftliche Verlagsgesellschaft mbH, Stuttgart, 1976, S. 15 ff. und S. 83 ff.

Unter den üblicherweise gewählten Oxalkylierungsbedingungen werden jedoch neben den eigentlichen Oxalkylierungsprodukten auch Nebenprodukte gebildet, deren Art, Zahl und Menge je nach gewählter Verfahrensvariante unterschiedlich sein kann. Häufig entstehen beispielsweise Carbonylverbindungen wie Acetaldehyd (in der Regel beim Einsatz von Ethylenoxid) oder höhere Aldehyde sowie deren Folgeprodukte, die Farbe und Geruch des Oxalkylierungsproduktes beeinflussen können.

Die gemäß den im Stand der Technik beschriebenen Ausführungsformen erhältlichen, oxalkylierten Amine und Alkohole, insbesondere die oben genannten Polyethylenimine, können daher je nach Oxalkylierungsgrad stark verfärbt sein und einen äußerst unangenehmen Geruch aufweisen, oder es muß unter unökonomischen Verfahrensbedingungen gearbeitet werden, beispielsweise mit großen Katalysatormengen, um die Verfärbung zu reduzieren.

Auch der Versuch, die Verfärbungen und geruchliche Belästigung verursachenden Verbindungen nachträglich wenigstens weitgehend zu entfernen, z. B. durch oxidative oder reduktive Bleichung oder durch Strippen, beispielsweise mit Stickstoff oder Wasserdampf, führt in der Regel nicht zum gewünschten Erfolg.

Der vorliegenden Erfindung lag daher die Aufgabe zugrunde, ein Verfahren zur Oxalkylierung von Aminen oder Alkoholen oder deren Gemischen zur Verfügung zu stellen, das zu möglichst wenig verfärbten Produkten führt, die zudem noch einen möglichst geringen Anteil an belästigende Gerüche verursachenden Verbindungen aufweisen.

Gelöst wird die erfindungsgemäße Aufgabe, indem die Oxalkylierung in Gegenwart von Ameisensäure oder einem Salz der Ameisensäure, oder einem Gemisch aus zwei oder mehr davon, durchgeführt wird.

Gegenstand der Erfindung ist daher ein Verfahren zur Herstellung eines oxalkylierten Amins oder eines Gemischs aus zwei oder mehr oxalkylierten Aminen, oder eines oxalkylierten Alkohols oder eines Gemischs aus zwei oder mehr oxalkylierten Alkoholen oder eines Gemischs aus einem oder mehreren oxalkylierten Aminen und einem oder mehreren oxalkylierten Alkoholen, oder eines Gemischs aus einem oder mehreren oxalkylierten Aminen und einem oder mehreren oxalkylierten Alkoholen, bei dem ein Reaktionsgemisch, enthaltend ein Amin oder ein Gemisch aus zwei oder mehr Aminen, oder einen Alkohol oder ein Gemisch aus zwei oder mehr Alkoholen, oder ein Gemisch aus einem oder mehreren Aminen und einem oder mehreren Alkoholen, und ein Alkylenoxid, oder ein Gemisch aus zwei oder mehr Alkylenoxiden, in einer oder mehreren aufeinanderfolgenden Reaktionsstufen umgesetzt wird, wobei mindestens eine der Reaktionsstufen in Gegenwart eines basischen Katalysators abläuft, dadurch gekennzeichnet, daß bei mindestens einer der Reaktionsstufen Ameisensäure oder ein Salz der Ameisensäure, oder ein Gemisch aus zwei oder mehr davon, im Reaktionsgemisch vorliegt, wobei ein oxalkyliertes Reaktionsprodukt entsteht.

Unter dem Begriff "Reaktionsstufe" wird im erfindungsgemäßen Sinne ein Reaktionsablauf verstanden, nach dessen Abschluß ein isolierbares Produkt oder Zwischenprodukt vorliegt, das keinen weiteren Reaktionen (mit Ausnahme von gegebenenfalls mit geringer Geschwindigkeit ablaufenden Nebenreaktionen) unterliegt. Das erfindungsgemäße Verfahren kann daher beispielsweise in einer einzigen "Reaktionsstufe" ablaufen, unabhängig davon, wieviele einzelne Reaktionsschritte im Reaktionsgemisch tatsächlich ablaufen, wenn nach Abschluß der Reaktion das gewünschte Endprodukt vorliegt. Das erfindungsgemäße Verfahren kann jedoch auch in mehreren Stufen durchgeführt werden, d.h., einzelne, zum gewünschten Endprodukt führende Stufen können nacheinander so ausgeführt werden, daß nach Beendigung einer ersten Stufe eine zweite Stufe folgt, die, zeitlich darauffolgend, im gleichen oder in einem anderen Reaktionsgefäß durchgeführt werden kann. Gegebenenfalls können anschließend noch weitere Stufen folgen, bis das gewünschte Endprodukt vorliegt.

Im Rahmen des erfindungsgemäßen Verfahrens können beispielsweise beliebige Verbindungen, die wenigstens eine Aminogruppe tragen (Amine) einer Oxalkylierung unterzogen werden, wobei es sich bei den Aminen um Verbindungen handeln kann, die eine oder mehrere primäre oder sekundäre Aminogruppen, sowie gegebenenfalls zusätzlich eine oder mehrere tertiäre Aminogruppen aufweisen können. Ebensogut können Gemische aus zwei oder mehr solcher Verbindungen dem erfindungsgemäßen Verfahren unterzogen werden. Das erfindungsgemäße Verfahren ist demnach nicht auf die Umsetzung von Verbindungen mit lediglich einer Aminogruppe beschränkt, sondern es können beispielsweise auch Di- oder Polyamine eingesetzt werden. Geeignete Amine sind beispielsweise Ammoniak, Methylamin, Ethylamin, 1-Propylamin, 2-Propylamin, 1-Butylamin, 2-Butylamin, 1-Pentylamin, 2-Pentylamin, 3-Pentylamin, die entsprechenden isomeren Hexylamine, Heptylamine, Octylamine, Nonylamine, Decylamine sowie höhere lineare oder verzweigte Alkylamine, wie sie beispielsweise durch Aminierung von Fettalkoholen mit bis zu 24 Kohlenstoffatomen erhältlich sind. Ebenfalls geeignet sind die entsprechenden sekundären Amine der genannten Verbindungen, wie sie beispielsweise durch Monoalkylierung der genannten Verbindungen erhältlich sind. Beispiele hierfür sind Dimethylamin, N-Methylethylamin, N-Diethylamin, N-Methylpropylamin, N-Methylbutylamin, N-Methylpentylamin, N-Methylhexylamin und dergleichen.

Weiterhin geeignet sind Oligoamine, die mindestens zwei Aminogruppen pro Molekül aufweisen. Hierzu zählen beispielsweise Ethylendiamin, Propylendiamin, Butylendiamin, Pentamethylendiamin, Hexamethylendiamin, Heptamethylendiamin, Octamethylendiamin und dergleichen, wobei von dieser Aufzählung auch die jeweiligen Stellungsisomeren der genannten Verbindungen, sofern existent, umfaßt werden. Ebenfalls im Rahmen des erfindungsgemäßen Verfahrens einsetzbar sind Amine, die an einem Molekül sowohl primäre als auch sekundäre und gegebenenfalls zusätzlich eine oder mehrere tertiäre Aminogruppen aufweisen. Hierzu zählen beispielsweise N-Methylethylendiamin, N-Ethylethylendiamin, N-Methylpropylendiamin, N-Ethylpropylendiamin, N-Methylbutylendiamin, N-Ethylbutylendiamin, N-Methylpentamethylendiamin, N-Methylhexamethylendiamin und dergleichen, sowie deren höhere Homologen, beispielsweise N-monoalkylierte Diamine mit bis zu 26 Kohlenstoffatomen, die linear oder verzweigt sein können. Ebenfalls geeignet sind Amine, die über eine unterschiedliche Anzahl primärer, sekundärer sowie gegebenenfalls tertiärer Aminogruppen in einem Molekül verfügen. Hierzu zählen beispielsweise Diethylentriamin, Triethylentetramin, Tetraethylenpentamin, N-(3-Aminopropyl)ethylendiamin, N-(4-Aminobutyl)ethylendiamin, N-(4-Aminobutyl)propylendiamin und dergleichen.

Weitere geeignete Amine sind Verbindungen aus der Gruppe der Polyethylenimine mit einem Zahlenmittel des Molekulargewichts (M_{w}) von beispielsweise 250 bis 2.000.000, vorzugsweise etwa 580 bis etwa 10.000. Solche Polyethylenimine werden in der Regel durch Polymerisation von Ethylenimin in wäßrigem Medium in Gegenwart von sauren Katalysatoren hergestellt. Als saure Katalysatoren kommen beispielsweise Mineralsäuren wie Salzsäure, Schwefelsäure, Phosphorsäure, Bromwasserstoffsäure und Jodwasserstoffsäure sowie organische Säuren wie Ameisensäure, Essigsäure und Propionsäure, Amidosulfonsäure, p-Toluolsulfonsäure und Benzolsulfonsäure, Natriumhydrogensulfat, Kaliumhydrogensulfat, Additionsprodukte von Schwefelsäure an Ethylendiamin und Additionsprodukte von Kohlendioxid an Ethylendiamin in Betracht. Außerdem eignen sich Alkylierungsmittel wie Methylchlorid, Ethylchlorid, Propylchlorid, Laurylchlorid und Benzylchlorid, sowie Lewissäuren wie Bortrifluorid. Die Menge an sauren Katalysatoren, bezogen auf Ethylenimin, beträgt beispielsweise weniger als 1 Gew.-% und liegt vorzugsweise in dem Bereich von 0,01 bis 1 Gew.-%. In einer bevorzugten Ausführungsform der vorliegenden Erfindung wird Polyethylenimin mit einem Molekulargewicht M_{w} von 600 bis 6.000 einer erfindungsgemäßen Oxalkylierung unterworfen.

Besonders vorteilhaft ist es, wenn bereits bei der Herstellung des Polyethylenimins Ameisensäure eingesetzt wurde. In einer bevorzugten Ausführungsform der Erfindung wird daher Polyethylenimin mit einem Molekulargewicht M_{w} von 600 bis 6.000, das unter Verwendung von Ameisensäure hergestellt wurde, einer erfindungsgemäßen Oxalkylierung unterworfen.

Im Rahmen des erfindungsgemäßen Verfahrens können auch Verbindungen, die wenigstens eine OH-Gruppe tragen (Alkohole) einer Oxalkylierung unterzogen werden, wobei es sich bei den Alkoholen um Verbindungen handeln kann, die jeweils eine oder mehrere primäre oder sekundäre oder tertiäre OH-Gruppen, oder gleichzeitig zwei oder mehr der verschiedenen genannten OH-Gruppen, aufweisen können. Ebensogut können Gemische aus zwei oder mehr solcher Verbindungen dem erfindungsgemäßen Verfahren unterzogen werden. Das erfindungsgemäße Verfahren ist demnach nicht auf die Umsetzung von Verbindungen mit lediglich einer OH-Gruppe (Monoalkohole) beschränkt, sondern es können beispielsweise auch Di- oder Polyalkohole eingesetzt werden. Zum Einsatz im Rahmen der erfindungsgemäßen Verfahrens sind beispielsweise lineare, verzweigte oder cyclische aliphatische C₁₋₄₄-Alkohole mit einer bis etwa zehn OH-Gruppen geeignet. Ebenso eignen sich mono- oder polycyclische, aromatische oder heteroaromatische C₆₋₄₀-Alkohole mit einer bis etwa zehn OH-Gruppen, wobei die aromatischen oder heteroaromatischen Alkohole beispielsweise aliphatische oder cycloaliphatische Substituenten aufweisen können oder Teile des Ringgerüsts cycloaliphatisch sein können. Gleichfalls geeignet sind beispielsweise OH-Gruppen tragende Polymere, wie sie beispielsweise durch Polymerisation, Polyaddition oder Polykondensation erhältlich sind.

Beispiele für geeignete Monoalkohole sind Methanol, Ethanol, Propanol, Isopropanol, 1-Butanol, 2-Butanol, tert.-Butanol, die isomeren Pentanole, Hexanole, Heptanole, Octanole, beispielsweise 2-Ethylhexanol, die linearen und verzweigten C₉₋₂₄-Fettalkohole, wie sie beispielsweise durch Oxo-Synthese erhältlich sind, cycloaliphatische Alkohole, beispielsweise Cyclohexanol, Cycloheptanol, Cyclooctanol, Hydroxymethylcyclohexan, Hydroxymethylcycloheptan, Hydroxymethylcyclooctan, Monohydroxyaromaten und substituierte Monohydroxyaromaten, beispielsweise Phenol, Methylphenol, Ethylphenol, Propylphenol, Butylphenol sowie deren Alkylhomologen, beispielsweise Octylphenol oder Nonylphenol, und dergleichen. Beispiele für geeignete Diole sind Ethylenglykol, 1,2-Propylenglykol, 1,3-Propylenglykol, 1,2-Butylenglykol, 1,3-Butylenglykol, 1,4-Butylenglykol, die isomeren Pentylenglykole und Hexylenglykole, beispielsweise 1,6-Hexandiol sowie deren höhere Homologen, o-, m- und p-Dihydroxybenzol, o-, m- und p-Bis(hydroxymethyl)benzol, 4,4'-Dihydroxybiphenyl, Bisphenol-A sowie die Produkte einer partiellen oder vollständigen Hydrierung der aromatischen Doppelbindungen der genannten Verbindungen.

Ebenso geeignet sind höhere Alkohole mit bis zu etwa 10 OH-Gruppen, insbesondere etwa 3 bis etwa 6 OH-Gruppen, beispielsweise Glycerin, Trimethylolpropan, Triethylolpropan, Pentaerythrit oder Kohlenhydrate wie Sorbit. Gleichfalls geeignet sind die Kondensationsprodukte (Mono- und Polyether) der genannten Verbindungen mit sich selbst oder von zwei oder mehr der genannten Verbindungen untereinander, mit bis zu etwa 20 oder 30 OH-Gruppen. Zu den OH-Gruppen tragenden Polymeren, wie sie im Rahmen der vorliegenden Erfindung eingesetzt werden können, zählen beispielsweise Polyvinylalkohol oder Hydroxygruppen tragende Polyacrylate, die etwa durch Homo- oder Copolymerisation von OH-Gruppen tragenden Acrylsäureestern erhältlich sind.

Der Begriff "Oxalkylierung" bezieht sich im Rahmen der vorliegenden Erfindung auf die Umsetzung eines der oben genannten Amine, oder eines Gemischs aus zwei oder mehr der oben genannten Amine, oder eines der oben genannten Alkohole oder eines Gemischs aus zwei oder mehr der oben genannten Alkohole oder eines Gemischs aus einem oder mehreren der oben genannten Amine und einem oder mehreren der oben genannten Alkohole mit einem Alkylenoxid, oder einem Gemisch aus zwei oder mehr verschiedenen Alkylenoxiden.

Bei den nach dem erfindungsgemäßen Verfahren zur Oxalkylierung einsetzbaren Alkylenoxiden handelt es sich vorzugsweise um Alkylenoxide der allgemeinen Formel I 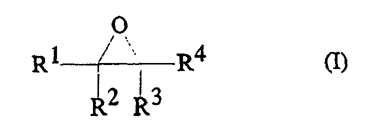 worin R¹, R², R³ und R⁴ gleich oder verschieden sind und jeweils unabhängig voneinander für Wasserstoff, C₁₋₁₀-Alkyl, C₂₋₁₀-Alkenyl, C₂₋₁₀-Alkinyl, C₃₋₁₀-Cycloalkyl, C₃₋₁₀-Cycloalkenyl, C₆₋₁₂-Aryl oder Heteroaryl stehen, wobei die Alkyl-, Alkenyl- oder Alkinylreste linear oder verzweigt sein können und ihrerseits noch weitere funktionelle Gruppen tragen können, und die Cycloalkyl-, Aryl- und Heteroarylreste ihrerseits noch weitere funktionelle Gruppen tragen können oder mit C₁₋₁₀-Alkyl-, Alkenyl-, Alkinyloder Arylresten substituiert sein können.

Bevorzugt eingesetzte Alkylenoxide der allgemeinen Formel I sind beispielsweise Ethylenoxid, Propylenoxid, Isobutylenoxid, 1,2-Butylenoxid, 2,3-Butylenoxid, Pentylenoxid oder Styroloxid, oder Gemische aus zwei oder mehr davon, wobei Ethylenoxid, Propylenoxid oder 1,2-Butylenoxid, oder Gemische aus zwei oder mehr davon, bevorzugt sind.

Das im Rahmen des vorliegenden Verfahrens einsetzbare Alkylenoxid, oder das Gemisch aus zwei oder mehr verschiedenen Alkylenoxiden, kann aus einer beliebigen Quelle, oder aus beliebigen unterschiedlichen Quellen stammen, d. h. nach irgendeinem beliebigen Verfahren hergestellt worden sein. Beispielsweise läßt sich Ethylenoxid durch katalytische Oxidation von Ethylen erhalten, wobei Ethylen und ein molekularen Sauerstoff enthaltendes Gas, beispielsweise Luft, mit Sauerstoff angereicherte Luft oder reiner Sauerstoff, in der Gasphase an einem silberhaltigen Katalysator zur Reaktion gebracht werden. Das im Rahmen der vorliegenden Erfindung einsetzbare Alkylenoxid, oder das Gemisch aus zwei oder mehr verschiedenen Alkylenoxiden, wird vorzugsweise in reiner Form eingesetzt. Das heißt, daß die eingesetzten Alkylenoxide im wesentlichen frei von Verunreinigungen sind, und damit im wesentlichen 100 % des Alkylenoxids, oder des Gemischs aus zwei oder mehr verschiedenen Alkylenoxiden, enthalten. Es ist jedoch ebenfalls möglich eine technische Qualität an Alkylenoxiden einzusetzen, die noch Verunreinigungen enthält, die üblicherweise vor der Aufreinigung des Alkylenoxids nach der Herstellung vorliegen.

Die Oxalkylierung kann nur mit einer einzigen Art von Alkylenoxid erfolgen, es kann sich jedoch auch um eine gemischte Oxalkylierung handeln. Setzt man beispielsweise ein Gemisch von zwei oder mehr verschiedenen Alkylenoxiden im Reaktionsgemisch ein, so führt das in der Regel, bei im wesentlichen vergleichbarer Reaktivität der Alkylenoxide, zu statistischen Polyetherketten, in denen die Abfolge der Gemischbestandteile keiner bestimmten Reihenfolge unterliegt. Führt man die verschiedenen Alkylenoxide dem Reaktionsgemisch jedoch nacheinander zu, d. h. wird ein weiteres, zur Umsetzung vorgesehenes, Alkylenoxid erst dann zugeführt, wenn das vorher zugeführte bereits vollständig umgesetzt ist, so lassen sich durch diese Verfahrensweise blockweise aufgebaute Polyethersegmente erhalten, in denen die Abfolge der einzelnen Alkylenoxidsegmente in der Polyetherkette in ihrer Reihenfolge und Länge von der Reihenfolge der Zugabe des jeweiligen Alkylenoxids und dessen Menge abhängig ist.

In einer bevorzugten Ausführungsform der Erfindung beträgt das molare Verhältnis von Alkylenoxidgruppen zu aciden, stickstoffgebundenen Wasserstoffatomen im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, etwa 1:1 bis etwa 300:1.

Als basischer Katalysator wird im Rahmen des erfindungsgemäßen Verfahrens in der Regel eine üblicherweise für basisch katalysierte Reaktionen verwendete, alkalisch wirkende Verbindung verwendet, beispielsweise Alkalimetallhydroxide wie Lithiumhydroxid, Natriumhydroxid, Kaliumhydroxid oder Cäsiumhydroxid, Alkalimetallalkoholate wie Natrium- oder Kaliummethanolat, Kaliumethanolat, Kaliumisopropylat oder Kaliumtertiärbutylat oder Gemische aus zwei oder mehr davon. Anstelle der genannten Kaliumalkoholate können auch die entsprechenden Natriumalkoholate eingesetzt werden. Weiterhin als basische Katalysatoren geeignet sind Natriumhydrid und heterogene Katalysatoren, beispielsweise Hydrotalcid, der gegebenenfalls modifiziert sein kann, oder deren Gemisch. Die Menge an basischem Katalysator im Reaktionsgemisch kann etwa 0,1 bis 20 mol-% betragen, bezogen auf acide, stickstoffgebundene Wasserstoffatome im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen. Vorzugsweise beträgt sie in der Regel etwa 1 bis etwa 10 mol-% und besonders bevorzugt etwa 2 bis etwa 5 mol-%.

Das erfindungsgemäße Verfahren kann in einer oder mehreren aufeinanderfolgenden Reaktionsstufen durchgeführt werden, wobei bei mindestens einer der Reaktionsstufen Ameisensäure oder das Salz der Ameisensäure, oder ein Gemisch aus zwei oder mehr davon, im Reaktionsgemisch vorliegt.

In einer besonders bevorzugten Ausführungsform liegt die Ameisensäure oder das Salz der Ameisensäure, oder ein Gemisch aus zwei oder mehr davon, bereits zu Beginn der Oxalkylierung im Reaktionsgemisch vor.

Als Salze der Ameisensäure sind grundsätzlich alle Salze geeignet, insbesondere eignen sich jedoch als Salze der Ameisensäure die Alkaliformiate, beispielsweise die Formiate des Lithiums, Natriums oder Kaliums, oder die Ammoniumformiate, wie sie beispielsweise aus Ameisensäure und Ammoniak oder organischen Aminen erhältlich sind. Besonders bevorzugt sind Natriumformiat und Kaliumformiat.

Die Ameisensäure oder das Salz der Ameisensäure, oder ein Gemisch aus zwei oder mehr davon, wird im Rahmen der erfindungsgemäßen Verfahrens in der Regel in einer Menge von etwa 0,1 bis etwa 10 mol-%, bezogen auf acide, stickstoffgebundene Wasserstoffatome im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, eingesetzt. In bevorzugten Ausführungsformen der Erfindung kommen beispielsweise Mengen von etwa 0,5 bis etwa 8 mol-%, beispielsweise etwa 1 bis etwa 6 mol-% oder etwa 2 bis etwa 5 mol-% zum Einsatz. Ebenfalls geeignet sind beispielsweise Mengen von etwa 3 bis etwa 4 mol-%.

Das erfindungsgemäße Verfahren kann beispielsweise einstufig durchgeführt werden. Hierbei wird ein Reaktionsgemisch, enthaltend ein Amin oder ein Gemisch aus zwei oder mehr Aminen, oder einen Alkohol oder ein Gemisch aus zwei oder mehr Alkoholen, oder ein Gemisch aus einem oder mehreren Aminen und einem oder mehreren Alkoholen, einen basischen Katalysator, Ameisensäure oder ein Salz der Ameisensäure oder ein Gemisch aus zwei oder mehr davon, sowie ein Alkylenoxid, oder ein Gemisch aus zwei oder mehr verschiedenen Alkylenoxiden, umgesetzt. Die Umsetzung erfolgt in einer bevorzugten Ausführungsform bei einer Temperatur von etwa 80°C bis etwa 170°C

Es ist bei einer einstufigen Reaktionsführung bevorzugt, wenn das Reaktionsgemisch im wesentlichen wasserfrei ist, d. h., daß der Wassergehalt des Reaktionsgemischs unterhalb von 1000 ppm, vorzugsweise unterhalb von 500 ppm liegt.

Um einen solchen Wassergehalt zu erzielen, können beispielsweise die einzelnen Bestandteile des Reaktionsgemischs schon in im wesentlichen wasserfreier Form eingesetzt werden. Es ist jedoch ebenso möglich, wenn beispielsweise eine oder mehrere der im Reaktionsgemisch vorliegenden Komponenten einen insgesamt zu hohen Wassergehalt aufweisen, das Reaktionsgemisch vor der Umsetzung von Wasser zu befreien. Dies kann beispielsweise dadurch geschehen, daß das Wasser vollständig aus dem Reaktionsgemisch abdestilliert wird. Hierzu wird das Reaktionsgemisch in der Regel entweder vermindertem Druck oder erhöhter Temperatur oder vorzugsweise beidem gleichzeitig ausgesetzt, so daß im Reaktionsgemisch enthaltenes Wasser abdestilliert. Hierbei ist zu beachten, daß keine der weiteren, im Reaktionsgemisch vorliegenden und zur Umsetzung vorgesehenen Komponenten zusammen mit dem Wasser abdestilliert. Sind im Reaktionsgemisch beispielsweise Komponenten vorhanden deren Siedepunkt unterhalb dem von Wasser liegt oder die mit Wasser zusammen ein Azeotrop bilden, so empfiehlt es sich, diese Verbindungen in möglichst wasserfreier Form einzusetzen und gegebenenfalls im Reaktionsgemisch vorhandenes Wasser vor deren Zugabe auf die beschriebene Art und Weise zu entfernen. Die Wasserentfernung kann beispielsweise auch mit Hilfe einer azeotropen Destillation erfolgen, indem man ein Schleppmittel wie Benzol, Toluol oder Xylol zusetzt und das Wasser azeotrop entfernt. Das zugesetzte Schleppmittel kann anschließend, bei vermindertem Druck, abdestilliert werden oder während der Oxalkylierung im Reaktionsgemisch verbleiben.

Bei der einstufigen Durchführung des erfindungsgemäßen Verfahrens beträgt die Reaktionstemperatur vorzugsweise etwa 100 bis 160°C, beispielsweise etwa 120 bis etwa 135°C oder etwa 135 bis etwa 150°C. Die Reaktionsdauer liegt in der Regel zwischen etwa 4 und etwa 20 h, beispielsweise bei etwa 8 bis etwa 12 h.

Die einstufige Verfahrensweise wird in einer bevorzugten Ausführungsform der Erfindung insbesondere bei der Oxalkylierung von Alkoholen angewandt.

In einer weiteren bevorzugten Ausführungsform der Erfindung wird die Reaktion zweistufig durchgeführt, wobei
a) in einer ersten Stufe ein Reaktionsgemisch, enthaltend ein Amin oder ein Gemisch aus zwei oder mehr Aminen, oder einen Alkohol oder ein Gemisch aus zwei oder mehr Alkoholen, oder ein Gemisch aus einem oder mehreren Aminen und einem oder mehreren Alkoholen, Ameisensäure oder ein Salz der Ameisensäure, oder ein Gemisch aus zwei oder mehr davon, sowie ein Alkylenoxid, oder ein Gemisch aus zwei oder mehr verschiedenen Alkylenoxiden, umgesetzt wird, wobei ein Reaktionsprodukt der ersten Stufe entsteht, und
b) in einer zweiten Stufe ein Reaktionsgemisch, enthaltend das Reaktionsprodukt der ersten Stufe, einen basischen Katalysator sowie ein Alkylenoxid, oder ein Gemisch aus zwei oder mehr verschiedenen Alkylenoxiden, umgesetzt wird.

Die Reaktionstemperatur beträgt in der ersten Stufe beispielsweise etwa 80°C bis etwa 130°C, in der zweiten Stufe beispielsweise mehr als etwa 130°C bis etwa 170°C.

Die zweistufigen Durchführung des erfindungsgemäßen Verfahrens wird insbesondere dann angewandt, wenn das Reaktionsgemisch ein Amin oder ein Gemisch aus zwei oder mehr Aminen enthält.

Wenn im Rahmen des erfindungsgemäßen Verfahrens ein Amin oder ein Gemisch aus zwei oder mehr Aminen umgesetzt werden soll, so enthält das Reaktionsgemisch in einer bevorzugten Ausführungsform der Erfindung in der ersten Stufe das Amin oder das Gemisch aus zwei oder mehr Aminen als wäßrige Lösung. Hierbei ist es bevorzugt, wenn das Amin oder das Gemisch aus zwei oder mehr Aminen in der wäßrigen Lösung in einer Konzentration von etwa 20 Gew.-% bis etwa 80 Gew.-%, insbesondere etwa 40 Gew.-% bis etwa 60 Gew.-%, vorliegt. Die weiteren, im Reaktionsgemisch der ersten Stufe vorliegenden Komponenten können ebenfalls in Form ihrer wäßrigen Lösungen eingesetzt werden, beispielsweise kann der basische Katalysator als etwa 20 bis etwa 80 gew.-%ige, vorzugsweise etwa 40 bis etwa 60 gew.-%ige, Lösung eingesetzt werden. Gleiches gilt selbstverständlich auch für die Ameisensäure oder das Salz der Ameisensäure, oder das Gemisch aus zwei oder mehr davon. In einer bevorzugten Ausführungsform der Erfindung wird vor Durchführung der zweiten Stufe Wasser im wesentlichen vollständig aus dem Reaktionsprodukt der ersten Stufe entfernt.

Der Einsatz wenigstens einer der im Reaktionsgemisch vorliegenden Komponenten als wäßrige Lösung ist dann vorteilhaft, wenn alle Komponenten des Reaktionsgemischs wasserlöslich sind. Wenn wenigstens eine der im Reaktionsgemisch vorliegenden Komponenten nicht über ausreichende Wasserlöslichkeit verfügt, so ist es in der Regel bevorzugt, wenn beim erfindungsgemäßen Verfahren nicht mit wäßrigen Lösungen gearbeitet wird. Eine ausreichende Wasserlöslichkeit einer Komponente liegt dann vor, wenn die Komponente sich zum wenigstens teilweise, vorzugsweise zu einem überwiegenden Teil, bei der Reaktionstemperatur in Wasser löst.

Gegebenenfalls kann das erfindungsgemäße Verfahren auch in organischen Lösemitteln oder ganz ohne Lösemittel durchgeführt werden. Als organische Lösemittel sind insbesondere alle polaren, aprotischen Lösemittel, beispielsweise Dimethylformamid (DMF) oder Dimethylacetamid (DMAc) geeignet, vorzugsweise wird jedoch auf den Einsatz von Lösemitteln verzichtet. Der Verzicht auf Lösemittel bietet sich insbesondere dann an, wenn die zu oxalkylierende Verbindung oder das Gemisch aus zu oxalkylierenden Verbindungen bei der Reaktionstemperatur in flüssigem Zustand vorliegt.

Die Reaktionstemperatur in der ersten Stufe beträgt etwa 80 bis etwa 130°C, vorzugsweise etwa 80 bis etwa 100°C. Die Reaktionsdauer der ersten Stufe beträgt beispielsweise etwa 2 bis etwa 12 h, wobei eine Reaktionsdauer von etwa 4 bis etwa 8 h bevorzugt ist.

Das molare Verhältnis von Alkylenoxidgruppen zu aciden, stickstoffgebundenen Wasserstoffatomen im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, kann in der ersten Stufe beispielsweise etwa 1 : 1 betragen. Es hat sich jedoch gezeigt, daß es sich auf die Farbe des entstehenden Produkts zusätzlich vorteilhaft auswirkt, wenn ein Verhältnis von weniger als 1, beispielsweise etwa 0,4 : 1 bis etwa 0,99 : 1, besonders bevorzugt etwa 0,6 : 1 bis etwa 0,9 : 1 gewählt wird.

In der ersten Stufe kann unter Normaldruck gearbeitet werden, gegebenenfalls kann die Reaktion jedoch auch in einem Autoklaven bei Drücken von bis zu etwa 20 bar erfolgen.

In der zweiten Stufe wird ein Reaktionsgemisch, enthaltend das Reaktionsprodukt der ersten Stufe, einen basischen Katalysator sowie ein Alkylenoxid, oder ein Gemisch aus zwei oder mehr verschiedenen Alkylenoxiden, bei einer Temperatur von mehr als 130°C bis etwa 170°C umgesetzt. Zur Vorbereitung der zweiten Stufe kann man beispielsweise so vorgehen, daß zunächst das in der ersten Verfahrensstufe erhaltene Reaktionsprodukt zur Trockne eingedampft wird, sofern ein Lösemittel verwendet wurde und das Reaktionsprodukt selbst nicht flüssig ist, und anschließend der basische Katalysator zugefügt wird.

Wenn in der ersten Verfahrensstufe in wäßriger Lösung gearbeitet wurde, ist insbesondere bevorzugt, zum in der ersten Reaktionsstufe erhaltenen, wäßrigen Reaktionsprodukt den basischen Katalysator zu geben und anschließend das Wasser, z. B. durch Destillieren unter vermindertem Druck oder mit Hilfe einer azeotropen Destillation, indem man beispielsweise ein Schleppmittel wie Benzol, Toluol oder Xylol zusetzt und das Wasserazeotrop entfernt, zu entfernen.

Die Oxalkylierung in der zweiten Verfahrensstufe wird bei Temperaturen von mehr als etwa 130°C bis etwa 170°C, vorzugsweise bei Temperaturen im Bereich von mehr als etwa 130°C bis etwa 145°C durchgeführt. Die Reaktion kann unter Atmosphärendruck oder vorzugsweise unter erhöhtem Druck erfolgen. Sie wird vorzugsweise in einem mit einem Rührer ausgestatteten Autoklaven bei Drücken von etwa 1 bis etwa 20, vorzugsweise bei etwa 2 bis etwa 10 bar, durchgeführt.

Die Menge an Alkylenoxid, oder an Gemisch aus zwei oder mehr verschiedenen Alkylenoxiden, wird in der zweiten Stufe so eingestellt, daß ein oxalkyliertes Amin oder ein Gemisch aus zwei oder mehr oxalkylierten Aminen, oder ein oxalkylierter Alkohol oder ein Gemisch aus zwei oder mehr oxalkylierten Alkoholen, oder ein Gemisch aus einem oder mehreren oxalkylierten Aminen und einem oder mehreren oxalkylierten Alkoholen entsteht, das etwa 1 bis etwa 200 mol Alkylenoxidgruppen pro acidem, stickstoffgebundenem Wasserstoffatom im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, aufweist.

Vorzugsweise enthalten die in der zweiten Verfahrensstufe entstehenden oxalkylierten Produkte etwa 1 bis etwa 20 mol umgesetztes Alkylenoxid, oder ein Gemisch aus zwei oder mehr verschiedenen umgesetzten Alkylenoxiden, pro acidem, stickstoffgebundenem Wasserstoffatom im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen. Die Reaktionsdauer in der zweiten Verfahrensstufe beträgt etwa 2 bis etwa 15 h und liegt vorzugsweise in einem Bereich von etwa 5 bis etwa 12 h.

In einer bevorzugten Ausführungsform der Erfindung wird das oxalkylierte Reaktionsprodukt nach Abschluß der Umsetzung für einen Zeitraum von 5 Minuten bis 5 Stunden bei einer Temperatur von 40°C bis 140°C bei einem Druck von 0,1 bis 100 mbar gehalten, wobei beispielsweise leichtflüchtige Verbindungen entfernt werden können.

Nach dem erfindungsgemäßen Verfahren erhält man in der Regel oxalkylierte Produkte, die gegenüber den aus dem Stand der Technik bekannten Produkten eine hellere Farbe und einen besseren Geruch aufweisen, wobei die im Vergleich zu den aus dem Stand der Technik bekannten Verfahren benötigte Katalysatormenge durch den erfindungsgemäßen Einsatz von Ameisensäure bzw. Salzen der Ameisensäure deutlich verringert werden kann.

Die Erfindung wird nachfolgend durch Beispiele erläutert, die jedoch keine beschränkende Wirkung auf den Gegenstand der Erfindung ausüben.

### Beispiele

### Beispiel 1:

### 1. Stufe:

1.470 g einer wäßrigen, 50 %igen Lösung eines Polyethylenimins (entsprechend 17,1 mol aciden, stickstoffgebundenen Wasserstoffatomen) wurden in einem Edelstahlautoklaven mit ca. 20 1 Volumen vorgelegt. Anschließend wurden 8 g Ameisensäure zugegeben. Der Reaktor wurde verschlossen und mit Stickstoff gespült. Es wurde nun unter Rühren auf 100°C erwärmt und bei dieser Temperatur wurden 748 g (17 mol) Ethylenoxid im Verlauf von 2 bis 3 h bei einem maximalen Druck von 5 bar zugegeben. Nach beendeter Zugabe und Erreichen eines konstanten Drucks wurde das Reaktionsgemisch noch 2 h bei 100°C gehalten, anschließend wurde auf 50°C abgekühlt und entspannt.

### 2. Stufe:

Das aus der ersten Stufe erhaltene Reaktionsprodukt wurde im gleichen Reaktor mit 74 g 50 % iger, wäßriger KOH-Lösung versetzt. Der Reaktor wurde verschlossen, bis zu einem Druck von 20 mbar evakuiert und langsam auf 100°C erwärmt. Zur weitgehenden Entfernung des Wassers aus dem Reaktionsgemisch wurde der Reaktor 6 h unter diesen Bedingungen gehalten.

Anschließend wurde der Reaktor mit Stickstoff entspannt und im Verlauf. von etwa 10 h wurden bei maximal 5 bar Druck 14.300 g (325 mol) Ethylenoxid bei 140°C zudosiert. Nach beendeter Zugabe und Erreichen von Druckkonstanz wurden noch weitere 2 h bei 140°C gerührt. Anschließend wurde langsam auf Normaldruck entspannt und auf etwa 80°C abgekühlt.

Danach wurde der Reaktor auf etwa 20 bis 50 mbar evakuiert und 1 h unter diesen Bedingungen zur Entfernung flüchtiger Bestandteile belassen. Anschließend wurde auf Zimmertemperatur abgekühlt, entspannt und der Reaktor entleert. Die Ausbeute betrug 15.800 g Ethoxylat mit einem durchschnittlichen Ethoxylierungsgrad von etwa 20. Das erhaltene Produkt ist ein gelbes Öl, das bei Zimmertemperatur langsam erstarrt und einen schwachen Geruch aufweist. Die Farbzahl in 10 %iger wäßriger Lösung beträgt 2 (Jod-Farbzahl).

Führt man den Versuch ohne den erfindungsgemäßen Zusatz von Ameisensäure durch, so erhält man das Endprodukt als bernsteinfarbenes, langsam erstarrendes Öl, mit einer Jod-Farbzahl (10 %ig in Wasser) von 8. Es zeigt einen scharfen, sehr unangenehmen Geruch.

Noch deutlicher läßt sich der Geruch der beiden Produkte unterscheiden, wenn man eine 0,5- oder 1 %ige wäßrige Lösung abriecht. Während das erfindungsgemäße Produkt nur einen schwachen Eigengeruch aufweist, riecht das Vergleichsprodukt scharf und stechend.

### Beispiel 2:

### a) Vergleichsbeispiel nach herkömmlicher Verfahrensweise:

2300 g Isononylphenol wurden in einem für Oxalkylierungen geeigneten Reaktor zusammen mit 2,5 g Kaliumhydroxid vorgelegt. Der Reaktor wurde verschlossen und das Gemisch auf 140°C aufgeheizt, wobei gleichzeitig Vakuum angelegt wurde. Die Mischung wurde auf diese Weise bei 30 mbar für 2 Stunden getrocknet und anschließend mit Stickstoff entspannt.

Bei 140°C und einem Druck von etwa 5 bar wurden dann 2580 g Ethylenoxid im Verlauf von 3 bis 4 Stunden zudosiert. Nach Beendigung der Zudosierung wurde zur Vervollständigung der Umsetzung noch etwa 2 Stunden gerührt. Anschließend wurde der Ansatz zur Entfernung eventuell vorhandener flüchtiger Bestandteile bei etwa 20 bis 50 mbar und einer Temperatur von etwa 100 bis 140°C gehalten. Nach Abkühlen und Entleeren des Reaktors erhielt man etwa 4800 g des Ethoxylats mit einer Farbzahl von 67 (APHA) und einem deutlichen Aldehydgeruch.

### b) Erfindungsgemäßes Verfahren

Die in a) angegebenen Verfahrensparameter wurden identisch wiederholt, es wurde jedoch der Isononylphenol/Kaliumhydroxid-Mischung von Beginn an eine Menge von 2,5 g Kaliumformiat zugesetzt.

Das so erhältliche Oxalkylat war bereits im direkten Vergleich deutlich heller als das nach a) erhaltene. Es besaß eine Farbzahl von 35 (APHA) und einen nur noch schwach wahrnehmbaren Geruch.

## Patentansprüche

1. Verfahren zur Herstellung eines oxalkylierten Amins oder eines Gemischs aus zwei oder mehr oxalkylierten Aminen, oder eines oxalkylierten Alkohols oder eines Gemischs aus zwei oder mehr oxalkylierten Alkoholen, oder eines Gemischs aus einem oder mehreren oxalkylierten Aminen und einem oder mehreren oxalkylierten Alkoholen, bei dem ein Reaktionsgemisch, enthaltend ein Amin oder ein Gemisch aus zwei oder mehr Aminen, oder einen Alkohol oder ein Gemisch aus zwei oder mehr Alkoholen, oder ein Gemisch aus einem oder mehreren Aminen und einem oder mehreren Alkoholen, und ein Alkylenoxid, oder ein Gemisch aus zwei oder mehr Alkylenoxiden, in einer oder mehreren aufeinanderfolgenden Reaktionsstufen umgesetzt wird, wobei mindestens eine der Reaktionsstufen in Gegenwart eines basischen Katalysators abläuft, **dadurch gekennzeichnet, daß** bei mindestens einer der Reaktionsstufen Ameisensäure oder ein Salz der Ameisensäure, oder ein Gemisch aus zwei oder mehr davon, im Reaktionsgemisch vorliegt, wobei ein oxalkyliertes Reaktionsprodukt entsteht.

2. Verfahren nach Anspruch 1, **dadurch gekennzeichnet, daß** die Ameisensäure oder das Salz der Ameisensäure, oder ein Gemisch aus zwei oder mehr davon, zu Beginn der Oxalkylierung im Reaktionsgemisch vorliegt.

3. Verfahren nach Anspruch 1 oder 2, **dadurch gekennzeichnet, daß** die Ameisensäure oder das Salz der Ameisensäure, oder ein Gemisch aus zwei oder mehr davon, in einer Menge von 0,1 bis 10 mol-%, bezogen auf die Gesamtmenge an aciden, stickstoffgebundenen Wasserstoffatomen im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, eingesetzt wird.

4. Verfahren nach einem der Ansprüche 1 bis 3, **dadurch gekennzeichnet, daß** das molare Verhältnis von Alkylenoxidgruppen zu aciden, stickstoffgebundenen Wasserstoffatomen im Amin oder im Gemisch aus zwei oder mehr Aminen oder im Alkohol oder im Gemisch aus zwei oder mehr Alkoholen oder im Gemisch aus einem oder mehreren Aminen und einem oder mehreren Alkoholen, 1:1 bis 300:1 beträgt.

5. Verfahren nach einem der Ansprüche 1 bis 4, **dadurch gekennzeichnet, daß** die Reaktion einstufig durchgeführt wird, wobei ein Reaktionsgemisch, enthaltend ein Amin oder ein Gemisch aus zwei oder mehr Aminen, oder einen Alkohol oder ein Gemisch aus zwei oder mehr Alkoholen, oder ein Gemisch aus einem oder mehreren Aminen und einem oder mehreren Alkoholen, einen basischen Katalysator, Ameisensäure oder ein Salz der Ameisensäure oder ein Gemisch aus zwei oder mehr davon, sowie ein Alkylenoxid, oder ein Gemisch aus zwei oder mehr verschiedenen Alkylenoxiden, umgesetzt wird.

6. Verfahren nach einem der Ansprüche 1 bis 4, **dadurch gekennzeichnet, daß** die Reaktion zweistufig durchgeführt wird, wobei
a) in einer ersten Stufe ein Reaktionsgemisch, enthaltend ein Amin oder ein Gemisch aus zwei oder mehr Aminen, oder einen Alkohol oder ein Gemisch aus zwei oder mehr Alkoholen, oder ein Gemisch aus einem oder mehreren Aminen und einem oder mehreren Alkoholen, Ameisensäure oder ein Salz der Ameisensäure, oder ein Gemisch aus zwei oder mehr davon, sowie ein Alkylenoxid, oder ein Gemisch aus zwei oder mehr verschiedenen Alkylenoxiden, umgesetzt wird, wobei ein Reaktionsprodukt der ersten Stufe entsteht, und
b) in einer zweiten Stufe ein Reaktionsgemisch, enthaltend das Reaktionsprodukt der ersten Stufe, einen basischen Katalysator sowie ein Alkylenoxid, oder ein Gemisch aus zwei oder mehr verschiedenen Alkylenoxiden, umgesetzt wird.

7. Verfahren nach Anspruch 6, **dadurch gekennzeichnet, daß** das Reaktionsgemisch in der ersten Stufe ein Amin oder ein Gemisch aus zwei oder mehr Aminen enthält, wobei das Amin oder das Gemisch aus zwei oder mehr Aminen als wäßrige Lösung vorliegt und vor der Durchführung der zweiten Stufe Wasser im wesentlichen vollständig aus dem Reaktionsprodukt der ersten Stufe entfernt wird.

8. Verfahren nach einem der Ansprüche 6 oder 7, **dadurch gekennzeichnet, daß** in der ersten Stufe das molare Verhältnis von Alkylenoxidgruppen zu aciden, stickstoffgebundenen Wasserstoffatomen im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, 0,6:1 bis 0,9:1 beträgt.

9. Verfahren nach einem der Ansprüche 1 bis 8, **dadurch gekennzeichnet, daß** das oxalkylierte Reaktionsprodukt nach Abschluß der Umsetzung für einen Zeitraum von 5 Minuten bis 5 Stunden bei einer Temperatur von 40°C bis 140°C bei einem Druck von 0,1 bis 100 mbar gehalten wird.

10. Verfahren nach einem der Ansprüche 1 bis 9, **dadurch gekennzeichnet, daß** die Menge an basischem Katalysator im Reaktionsgemisch 0,1 bis 20 mol-%, bezogen auf acide, stickstoffgebundene Wasserstoffatome im Amin oder im Gemisch aus zwei oder mehr Aminen, oder im Alkohol oder im Gemisch aus zwei oder mehr Alkoholen, oder im Gemisch aus einem oder mehreren Aminen und einem oder mehreren Alkoholen, beträgt.

## Claims

1. A process for preparing an alkoxylated amine or a mixture of two or more alkoxylated amines, or an alkoxylated alcohol or a mixture of two or more alkoxylated alcohols, or a mixture of one or more alkoxylated amines and one or more alkoxylated alcohols, in which a reaction mixture comprising an amine or a mixture of two or more amines, or an alcohol or a mixture of two or more alcohols, or a mixture of one or more amines and one or more alcohols, and an alkylene oxide or a mixture of two or more alkylene oxides is reacted in one or more successive reaction steps, wherein at least one of the reaction steps is carried out in the presence of a basic catalyst and wherein formic acid or a salt of formic acid or a mixture of two or more thereof is present in the reaction mixture in at least one of the reaction steps, so as to give an alkoxylated reaction product.

2. A process as claimed in claim 1, wherein the formic acid or the salt of formic acid, or a mixture of two or more thereof, is present in the reaction mixture at the beginning of the alkoxylation.

3. A process as claimed in claim 1 or 2, wherein the formic acid or the salt of formic acid, or a mixture of two or more thereof, is used in an amount of from 0.1 to 10 mol%, based on the total amount of acidic hydrogen atoms bound to nitrogen in the amine or in the mixture of two or more amines, or in the alcohol or in the mixture of two or more alcohols, or in the mixture of one or more amines and one or more alcohols.

4. A process as claimed in any of claims 1 to 3, wherein the molar ratio of alkylene oxide groups to acidic hydrogen atoms bound to nitrogen in the amine or in the mixture of two or more amines or in the alcohol or in the mixture of two or more alcohols or in the mixture of one or more amines and one or more alcohols is from 1:1 to 300:1.

5. A process as claimed in any of claims 1 to 4, wherein the reaction is carried out in one step by reacting a reaction mixture comprising an amine or a mixture of two or more amines, or an alcohol or a mixture of two or more alcohols, or a mixture of one or more amines and one or more alcohols, a basic catalyst, formic acid or a salt of formic acid or a mixture of two or more thereof, together with an alkylene oxide or a mixture of two or more different alkylene oxides.

6. A process as claimed in any of claims 1 to 4, wherein the reaction is carried out in two steps, where
a) in a first step, a reaction mixture comprising an amine or a mixture of two or more amines, or an alcohol or a mixture of two or more alcohols, or a mixture of one or more amines and one or more alcohols, formic acid or a salt of formic acid, or a mixture of two or more thereof, together with an alkylene oxide, or a mixture of two or more different alkylene oxides, is reacted to form a reaction product of the first step, and
b) in a second step, a reaction mixture comprising the reaction product of the first step, a basic catalyst and an alkylene oxide, or a mixture of two or more different alkylene oxides, is reacted.

7. A process as claimed in claim 6, wherein the reaction mixture in the first step comprises an amine or a mixture of two or more amines, where the amine or the mixture of two or more amines is present as an aqueous solution and water is removed essentially completely from the reaction product of the first step before the second step is carried out.

8. A process as claimed in claim 6 or 7, wherein the molar ratio of alkylene oxide groups to acidic hydrogen atoms bound to nitrogen in the amine or in the mixture of two or more amines, or in the alcohol or in the mixture of two or more alcohols, or in the mixture of one or more amines and one or more alcohols, in the first step is from 0.6:1 to 0.9:1.

9. A process as claimed in any of claims 1 to 8, wherein the alkoxylated reaction product is maintained at from 40°C to 140°C and a pressure of from 0.1 to 100 mbar for a period of from 5 minutes to 5 hours after conclusion of the reaction.

10. A process as claimed in any of claims 1 to 9, wherein the amount of basic catalyst in the reaction mixture is from 0.1 to 20 mol%, based on acidic hydrogen atoms bound to nitrogen in the amine or in the mixture of two or more amines, or in the alcohol or in the mixture of two or more alcohols, or in the mixture of one or more amines and one or more alcohols.

## Revendications

1. Procédé de préparation d'une amine alcoxylée ou d'un mélange de deux ou de plusieurs amines alcoxylées, ou d'un alcool alcoxylé ou d'un mélange de deux ou plusieurs alcools alcoxylés, ou d'un mélange d'une ou plusieurs amines alcoxylées et d'un ou plusieurs alcools alcoxylés, dans lequel on fait réagir un mélange réactionnel contenant une amine ou un mélange de deux ou plusieurs amines, ou un alcool ou un mélange de deux ou plusieurs alcools, ou un mélange d'une ou plusieurs amines et d'un ou plusieurs alcools, avec un oxyde d'alkylène ou un mélange de deux ou plusieurs oxydes d'alkylène dans une ou plusieurs étapes de réaction successives, au moins une des étapes de réaction se déroulant en présence d'un catalyseur basique, **caractérisé en ce que**, dans au moins une des étapes de réaction, de l'acide formique ou un sel de l'acide formique ou un mélange de deux ou de plusieurs sels de celui-ci est présent dans le mélange réactionnel, produisant un produit réactionnel alcoxylé.

2. Procédé selon la revendication 1, **caractérisé en ce que** l'acide formique ou le sel de l'acide formique, ou un mélange de deux ou de plusieurs sels de celui-ci est présent dans le mélange réactionnel au début de l'alcoxylation.

3. Procédé selon la revendication 1 ou 2, **caractérisé en ce que** l'on ajoute l'acide formique ou le sel de l'acide formique, ou un mélange de deux ou plusieurs sels de celui-ci, à raison de 0,1% à 10% en moles, par rapport à la quantité totale d'atomes d'hydrogène acides liés à un atome d'azote dans l'amine ou le mélange de deux ou plusieurs amines, ou dans l'alcool ou le mélange de deux ou plusieurs alcools, ou dans le mélange d'une ou de plusieurs amines et d'un ou de plusieurs alcools.

4. Procédé selon l'une quelconque des revendications 1 à 3, **caractérisé en ce que** le rapport molaire des groupes oxyde d'alkylène aux atomes d'hydrogène acides liés à un atome d'azote dans l'amine ou le mélange de deux ou plusieurs amines ou dans l'alcool ou le mélange de deux ou plusieurs alcools ou dans le mélange d'une ou plusieurs amines et d'un ou plusieurs alcools va de 1:1 à 300:1.

5. Procédé selon l'une quelconque des revendications 1 à 4, **caractérisé en ce que** l'on réalise la réaction en une étape, le mélange réactionnel contenant une amine ou un mélange de deux ou plusieurs amines, ou un alcool ou un mélange de deux ou plusieurs alcools, ou un mélange d'une ou plusieurs amines et d'un ou plusieurs alcools, un catalyseur basique, de l'acide formique ou un sel de l'acide formique ou un mélange de deux ou plusieurs sels de celui-ci, ainsi qu'un oxyde d'alkylène ou un mélange de deux ou plusieurs oxydes d'alkylène différents.

6. Procédé selon l'une quelconque des revendications 1 à 4, **caractérisé en ce que** l'on réalise la réaction en deux étapes, dans lequel
a) dans une première étape, on fait réagir un mélange réactionnel contenant une amine ou un mélange de deux ou plusieurs amines, ou un alcool ou un mélange de deux ou plusieurs alcools, ou un mélange d'une ou plusieurs amines et d'un ou plusieurs alcools, de l'acide formique ou un sel de l'acide formique ou un mélange de deux ou plusieurs sels de celui-ci, ainsi qu'un oxyde d'alkylène ou un mélange de deux ou plusieurs oxydes d'alkylène différents, produisant ainsi le produit réactionnel de la première étape, et
b) dans une deuxième étape, on fait réagir un mélange réactionnel contenant le produit réactionnel de la première étape, un catalyseur basique ainsi qu'un oxyde d'alkylène ou un mélange de deux ou plusieurs oxydes d'alkylène différents.

7. Procédé selon la revendication 6, **caractérisé en ce que** le mélange réactionnel dans la première étape, contient une amine ou un mélange de deux ou plusieurs amines, dans lequel l'amine ou le mélange de deux ou plusieurs amines est présent sous forme de solution aqueuse, et l'eau, avant la réalisation de la deuxième étape, est presque entièrement éliminée du produit réactionnel.

8. Procédé selon l'une quelconque des revendications 6 ou 7, **caractérisé en ce que** dans la première étape, le rapport molaire des groupes oxyde d'alkylène aux atomes d'hydrogène acides liés à un atome d'azote dans l'amine ou le mélange de deux ou plusieurs amines, ou dans l'alcool ou le mélange de deux ou plusieurs alcools, ou dans le mélange d'une ou plusieurs amines et d'un ou plusieurs alcools va de 0,6:1 à 0,9:1.

9. Procédé selon l'une quelconque des revendications 1 à 8, **caractérisé en ce que** le produit réactionnel alcoxylé est maintenu après la fin de la réaction pendant une durée de 5 minutes à 5 heures à une température de 40°C à 140°C, sous une pression de 0,1 à 100 mbars.

10. Procédé selon l'une quelconque des revendications 1 à 9, **caractérisé en ce que** la quantité de catalyseur basique dans le mélange réactionnel est de 0,1% à 20% en moles, par rapport aux atomes d'hydrogène acides liés à un atome d'azote dans l'amine ou le mélange de deux ou plusieurs amines, ou dans l'alcool ou le mélange de deux ou plusieurs alcools, ou dans le mélange d'une ou plusieurs amines et d'un ou plusieurs alcools.
